(19) **Europäisches Patentamt**
**European Patent Office**
**Office européen des brevets**

(11) Publication number: **0 326 508 B1**

# (12) EUROPEAN PATENT SPECIFICATION

(45) Date of publication of patent specification:
08.01.92 Bulletin 92/02

(51) Int. Cl.$^5$: **F01D 5/18, B23P 15/02**

(21) Application number: **89630017.5**

(22) Date of filing: **24.01.89**

(54) **Method of fabricating an air cooled turbine blade.**

(30) Priority: **25.01.88 US 147464**

(43) Date of publication of application:
**02.08.89 Bulletin 89/31**

(45) Publication of the grant of the patent:
**08.01.92 Bulletin 92/02**

(84) Designated Contracting States:
**DE FR GB**

(56) References cited:
EP-A- 0 227 577
EP-A- 0 227 580
EP-A- 0 228 338
FR-A- 963 824
FR-A- 1 147 239
GB-A- 435 906
US-A- 4 672 727

(73) Proprietor: **UNITED TECHNOLOGIES CORPORATION**
**United Technologies Building 1, Financial Plaza**
**Hartford, CT 06101 (US)**

(72) Inventor: **Hall, Kenneth B.**
**13432 150th Court North**
**Jupiter Florida 33478 (US)**
Inventor: **Landis, Kenneth K.**
**152 Fairview East**
**Tequesta Florida 33469 (US)**

(74) Representative: **Weydert, Robert et al**
**OFFICE DENNEMEYER S.à.r.l. P.O. Box 1502**
**L-1015 Luxembourg (LU)**

EP 0 326 508 B1

## Description

The invention relates to a method of manufacturing air cooled turbine blades and in particular to a method of forming cooling air slots.

In gas turbines one of the limitations on gas temperature, and therefore efficiency of the gas turbine engine, is the ability of turbine blades to endure the high gas temperatures. It is accordingly known to cool the external surface of airfoils by conducting cooling air through an internal cavity in the blade and through a plurality of small passages discharging the air. It is desirable that the air exiting from these passages remain entrained in the boundary layer on the surface of the airfoil for as long a distance as possible downstream of the passage providing a protecting film of cool air between the hot mainstream gas and the airfoil surface. This use of cooling air itself decreases engine efficiency and therefore it is desirable to use as small an amount as possible of cooling air. Accordingly, the designer is challenged with obtaining maximum cooling with a limited amount of air.

Typically the airflow is metered by small metering openings at the inlet to each air passageway. Since the available pressure differential is fixed by other features of the engine design, the flow is established by sizing these holes. Metering at this location also provides appropriate distribution between the various airfoil cooling slots.

The angle which the flow through the passage makes with the airfoil surface and its direction with respect to the hot gas flow are also important factors. It is generally known that the closer that this cooling air comes to being tangent with the surface, the better the cooling effectiveness.

An air cooled turbine blade manufactored from a casted airfoil blank as referred to in the precharacterizing portion of independent claim 1 is disclosed in US-A-4,672,727. The known blade has a plurality of metering holes in the inner surface substantially perpendicular thereto, each of which feeds a diverging slot. The diverging slot forms an angle which is recommended as less than 30 degrees with respect to the airfoil surface. While a small angle of the slot with respect to the airfoil outer surface is desired, practical difficulties in locating and machining the slots limit this angle. Reference is also made to EP-A-0227580 wherein a longitudinally extending slot is formed in the airfoil inner surface and a plurality of passages machined from the outside of the airfoil intersect the slot. The passages are usually formed by electro-discharge machinery using a "comb" electrode having a plurality of longitudinally extending teeth, note Fig. 8 of EP-A-0227577. When the electrode is moved into the airfoil wall the teeth form the passages. The electrode can be moved into the airfoil wall for a discharge beyond the length of the teeth to ultimately form a longitudinal groove in the airfoil outer surface and all of the passages then communicate with this groove inwardly of the outer airfoil surface. Again, practical difficulties in locating and machining the passages limits the angle between the passages and the airfoil outer surface.

It is therefore the object of the invention to provide a method whereby the slots may be accurately located and formed in a manner which approaches a straight angle with respect to the airfoil downstream surface.

In accordance with the invention, to achieve this, there is provided a method of manufacturing a hollow air cooled turbine blade having an airfoil with an outer surface and an inner surface defining a cooling air plenum, a plurality of outwardly extending indentations in said inner surface, and a longitudinally extending slot machined from the outside of said airfoil and having a depth to intersect said indentations, said method comprising casting an airfoil blank and being characterized in that prior to machining said slot a skewed V-shaped groove extending in longitudinal direction of said airfoil blank is formed on said outer surface at a location adjacent to said indentations, with a first groove surface being formed substantially perpendicular to said outer surface and a second groove surface being formed to extend from said first groove surface in a direction away from said indentations and to define an angle of less than 20° with said outer surface, and that, after forming said V-shaped groove, said slot is machined in said first groove surface to a width less than the width of said first groove surface and substantially parallel to and coextensive with said second groove surface.

The use of the groove facilitates machining and provides a precise manner for locating the slot even with an extremely shallow angle.

Advantageous method steps are recited in the dependent claims 2 through 9.

The method of manufacturing an air cooled turbine blade will now be described in greater detail with reference to the drawings, wherein :

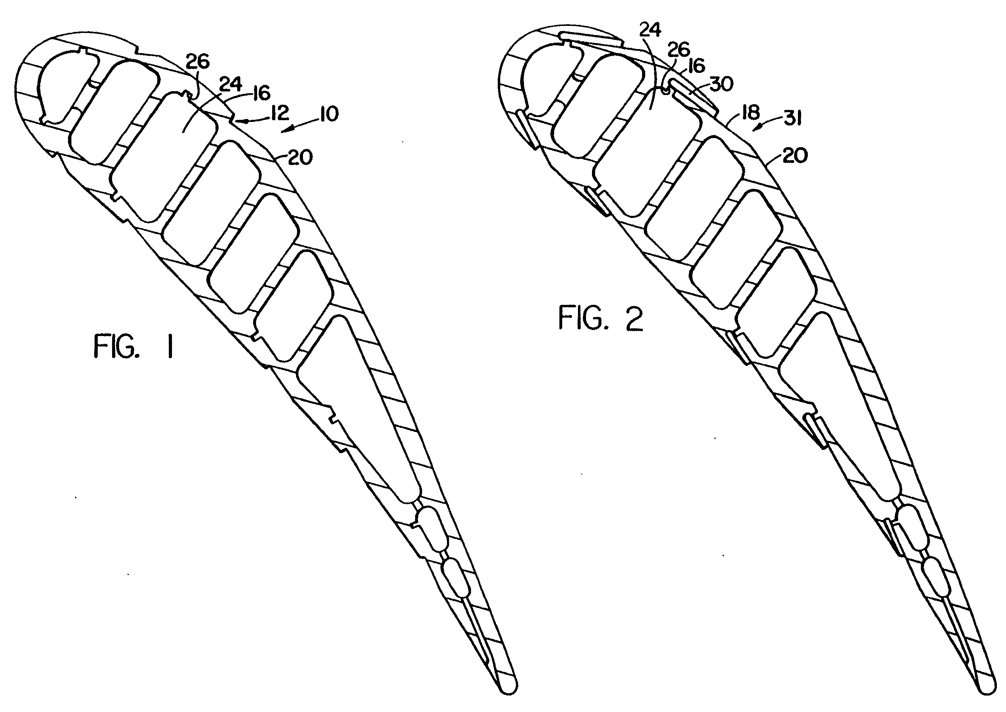

Figure 1 is a sectional view of the cast airfoil blank ;

Figure 2 is a sectional view of the final machined airfoil ;

Figure 3 is a detail of the area of a groove and indentation prior to machining ;

Figure 4 is a view similar to Figure 3 after machining ;

Figure 5 is a view of the area adjacent to the groove before machining including offset airfoil surfaces ; and

Figure 6 is a view similar to Figure 5 after machining.

An airfoil blank 10 is cast with a plurality of longitudinally extending grooves 12 having a first groove surface 14 which is substantially perpendicular to airfoil surface 16. A second groove surface 18 intersects downstream surface 20 at a small angle 22 which is as small as possible and preferably less than 20 degrees.

The airfoil blank includes a plurality of internal plenums 24 for the passage of cooling air. A plurality of discrete indentations 26 are cast in the inner surface of the airfoil at a spacing of about 1.3 mm (50 mils). These indentations may be circular or oval or any other shape, their purpose being predominantly to provide a metering flow passage to later formed slots.

Thereafter segmented slots 30 are electromachined into the blank forming airfoil 31. These slots are 4.3 mm (170 mils) in length with a 0.76 mm (30 mil) land 32 between adjacent slots. Land 32 improves the straightening of air flow and also acts to support the cantilever portion 34 of the airfoil blade.

The inner groove surface 36 is coextensive with second groove surface 18 to form a smooth flow path therein. It is preferable that the machining remove some of surface 18 rather than leave a step change at the interface.

The flow area of slot 30 is preferably four times the flow area of the total of holes 26 supplying the slot, with the holes 26 being 0.36 mm (14 mils) in diameter, while the slot is 0.2 mm (8 mils) wide by 4.3 mm (170 mils) long. A larger relative slot area than this results in decreased discharge velocity and a lack of appropriate distribution of the air flow through slots 26 along the length of slot 30. A lower ratio of flow area in the slot destroys the metering effect of this in hole 26.

The trailing edge 38 of cantilevered portion 34 should be as thin as possible since it is known that increased thickness induces turbulence which reduces the effectiveness of the downstream film cooling. Accordingly, with a first groove surface 14 of 0.25 mm (10 mils) a slot of 0.2 mm (8 mils) is selected leaving only 0.05 mm (2 mils) at the trailing edge 38. This preferably should be designed to be less than 0.076 mm (3 mils).

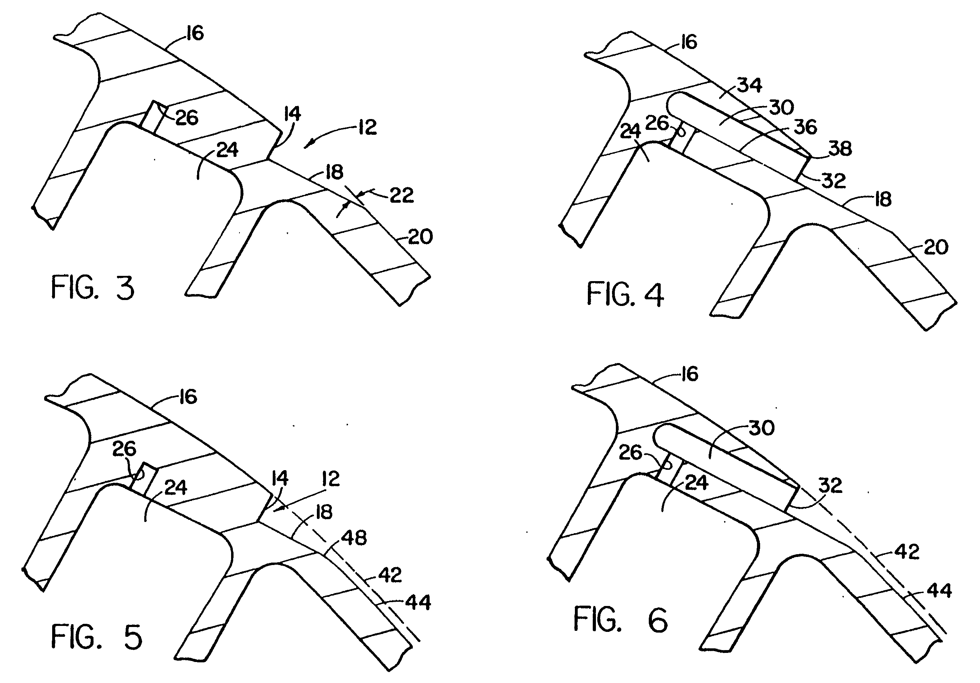

Figure 5 illustrates an embodiment where the airfoil surface 16 has a continuous extension 42 which is offset 0.076 mm (3 mils) from the downstream airfoil surface 44. This offset is related to the thickness of the entry cooling flow, and is preferably between 0.05 and 0.25 mm (2 and 10 mils).

A rounded edge 48 of 0.76 mm (30 mils) phases together the second groove surface 18 with the downstream surface 44. This rounded surface is preferably greater than 0.63 mm (25 mils) and may be easily cast the same time that groove 12 is. It provides an improved flowpath for the air film to stay against the downstream wall for cooling effectiveness. Furthermore, the offset between surface 16 and 44 provides a natural gap which is filled by this cooling air flow, and this step change is easily cast together with the groove.

In a manner similar to Figure 4 the groove 30 is machined between lands 32 to form the final airfoil structure.

## Claims

1. Method of manufacturing a hollow air cooled turbine blade having an airfoil with an outer surface (16, 20 ; 44) and an inner surface defining a cooling air plenum (24), a plurality of outwardly extending indentations (26) in said inner surface, and a longitudinally extending slot (30) machined from the outside of said airfoil and having a depth to intersect said indentations (26), said method comprising casting an airfoil blank (10) and being characterized in that prior to machining said slot (30) a skewed V-shaped groove (12) extending in longitudinal direction of said airfoil blank (10) is formed on said outer surface (16, 20 ; 44) at a location adjacent to said indentations (26), with a first groove surface (14) being formed substantially perpendicular to said outer surface (16, 20 ; 44) and a second groove surface (18) being formed to extend from said first groove surface (14) in a direction away from said indentations (26) and to define an angle (22) of less than 20° with said outer surface (16, 20 ; 44), and that, after forming said V-shaped groove (12), said slot (30) is machined in said first groove surface (14) to a width less than the width of said first groove surface (14) and substantially parallel to and coextensive with said second groove surface (18).

2. Method according to claim 1, characterized by rounding the edge (48) of the intersection of said second groove surface (18) with said outer surface (44).

3. Method according to claim 2, characterized by rounding said edge (48) of the intersection between said second groove surface (18) and said outer surface (44) to a radius greater than 0.63 mm (25 mils).

4. Method according to claim 1, characterized in that said longitudinally extending skewed V-groove (12) on said outer surface (16, 20 ; 44) is formed during casting of said airfoil blank (10).

5. Method according to claim 1, characterized in that the plurality of outwardly extending indentations (26) are formed during casting of said airfoil blank.

6. Method according to claim 1, characterized in that said longitudinally extending slot (30) is formed by machining the slot (30) in a plurality of longitudinally extending slot segments leaving a land (32) of blade material between adjacent segments.

7. Method according to claim 1, characterized in that said longitudinally extending slot (30) is formed by machining said slot (30) of a width with respect to the depth of said first groove surface (14) within 0.076 mm (3 mils) of said outer surface (16) whereby the edge of the remaining material does not exceed 0.076 mm (3 mils) in thickness.

8. Method according to claim 1, characterized by casting said airfoil blank (10) with a step change from a hypothetical continuous extension (42) of said airfoil surface (16) at the location of said groove (12).

9. Method according to claim 8, characterized by forming said airfoil blank (10) with a step change between 0.05 and 0.25 mm (2 and 10 mils).


## Revendications

1. Procédé de fabrication d'une aube de turbine creuse refroidie à l'air, comportant un profil aérodynamique ayant une surface externe (16, 20 ; 44) et une surface interne définissant une chambre de tranquillisation (24) contenant de l'air de refroidissement, une pluralité d'indentations ou échancrures (26), débouchant vers l'extérieur, dans cette surface interne, et une fente (30), s'étendant longitudinalement, usinée à partir de l'extérieur du profil aérodynamique et ayant une profondeur suffisante pour recouper les indentations (26), ce procédé comprenant une étape de coulée d'une ébauche de profil aérodynamique (10) et étant caractérisé en ce qu'avant l'usinage de la fente (30), une gorge inclinée (12) en forme de V, s'étendant dans la direction longitudinale de l'ébauche (10) du profil aérodynamique, est formée dans la surface externe (16, 20 ; 44) en un emplacement voisin des indentations (26), une première surface (14) de la gorge étant formée de manière à être sensiblement perpendiculaire à la surface externe (16, 20 ; 44) tandis qu'une seconde surface (18) de la gorge est formée de manière à s'étendre, à partir de la première surface (14) de la gorge, à l'opposé des indentations (26) et à définir un angle (22) inférieur à 20° avec la surface externe (16, 20 ; 44) et en ce que, après la formation de la gorge (12) en forme de V, la fente (30) est usinée dans la première surface (14) de la gorge avec une largeur inférieure à la largeur de cette première surface (14) de la gorge, cette fente étant sensiblement parallèle à la seconde surface (18) de la gorge et coextensive avec celle-ci.

2. Procédé suivant la revendication 1 caractérisé en ce qu'on arrondit l'arrête (48) située à l'intersection de la seconde surface (18) de la gorge et de la surface externe (44).

3. Procédé suivant la revendication 2 caractérisé en ce qu'on arrondit l'arête (48) de l'intersection entre la seconde surface (18) de la gorge et la surface externe (44) de manière qu'elle ait un rayon supérieur à 0,63 millimètre (25 mils).

4. Procédé suivant la revendication 1 caractérisé en ce que la gorge inclinée en V (12), s'étendant longitudinalement sur la surface externe (16, 20 ; 44), est formée pendant la coulée de l'ébauche (10) du profil aérodynamique.

5. Procédé suivant la revendication 1 caractérisé en ce que la pluralité d'indentations (26) débouchant à l'extérieur sont formées pendant la coulée de l'ébauche du profil aérodynamique.

6. Procédé suivant la revendication 1 caractérisé en ce que la fente (30) s'étendant longitudinalement est formée en usinant la fente (30) sous la forme d'une pluralité de segments de fente s'étendant longitudinalement, en laissant une portée (32) de la matière de l'aube entre les segments voisins.

7. Procédé suivant la revendication 1 caractérisé en ce que la fente (30) s'étendant longitudinalement est formée en usinant cette fente (30) de manière qu'elle ait une largeur, par rapport à la profondeur de la première surface (14) de la gorge, telle qu'elle se trouve au plus à 0,076 millimètre (3 mils) de la surface externe (16), si bien que le bord de la matière restante a une épaisseur qui ne dépasse pas 0,076 millimètre (3 mils).

8. Procédé suivant la revendication 1 caractérisé en ce qu'on coule l'ébauche (10) du profil aérodynamique avec qu'une transition à gradin à partir d'une extension continue théorique (42) de la surface (16) du profil aérodynamique, à l'emplacement de la gorge (12).

9. Procédé suivant la revendication 8 caractérisé en ce qu'on forme l'ébauche (10) du profil aérodynamique avec une transition à gradin comprise entre 0,05 et 0,25 millimètre (entre 2 et 10 mils).


## Patentansprüche

1. Verfahren zum Herstellen einer hohlen, luftgekühlten Turbinenschaufel, die ein Schaufelblatt mit einer äußeren Oberfläche (16, 20 ; 44) und einer inneren Oberfläche, welche eine Kühlluftkammer (24) begrenzt, mehrere sich nach außen erstreckende Vertiefungen (26) in der inneren Oberfläche und einen sich in Längsrichtung erstreckenden Schlitz (30) hat, der in der Außenseite des Schaufelblattes spanabhebend hergestellt worden ist und eine derartige Tiefe hat, daß er die Vertiefungen (26) schneidet, wobei das Verfahren beinhaltet, einen Schaufelblattrohling (10) zu gießen, und dadurch gekennzeichnet ist, daß vor dem spanabhebenden Herstellen des Schlitzes (30) eine schräge V-förmige Nut (12), die sich in Längsrichtung des Schaufelblattrohlings (10) erstreckt, an der äußeren Oberfläche (16, 20 ; 44) an einer Stelle hergestellt wird, die den Vertiefungen (26) benachbart ist, wobei eine erste Nutoberfläche (14) im wesentlichen rechtwinkelig zu der äußeren Oberfläche (16, 20 ; 44) und eine zweite Nutoberfläche (18) so hergestellt wird, daß sie sich von der ersten Nutoberfläche (14) aus in Richtung weg von den Vertiefungen (26) erstreckt und einen Winkel (22) von weniger als 20° mit der äußeren Oberfläche (16, 20 ; 44) bildet, und daß nach dem Herstellen der V-förmigen Nut (12) der Schlitz (30) in der ersten Nutoberfläche (14) bis zu einer Breite hergestellt wird, die kleiner ist als die Breite der ersten Nutoberfläche (14), und im wesentlichen parallel zu der zweiten Nutoberfläche (18) und mit der gleichen Ausdehnung wie diese.

2. Verfahren nach Anspruch 1, gekennzeichnet durch Abrunden der Kante (48) des Schnittes der zweiten Nutoberfläche (18) mit der äußeren Oberfläche (44).

3. Verfahren nach Anspruch 2, gekennzeichnet durch Abrunden der Kante (48) des Schnittes zwischen der zweiten Nutoberfläche (18) und der äußeren Oberfläche (44) auf einen Radius von mehr als 0,63 mm (25 mils).

4. Verfahren nach Anspruch 1, dadurch gekennzeichnet, daß die sich in Längsrichtung erstreckende schräge V-Nut (12) an der äußeren Oberfläche (16, 20 ; 44) während des Gießens des Schaufelblattrohlings (10) gebildet wird.

5. Verfahren nach Anspruch 1, dadurch gekennzeichnet, daß die sich nach außen erstreckenden Vertiefungen (26) während des Gießens des Schaufelblattrohlings gebildet werden.

6. Verfahren nach Anspruch 1, dadurch gekennzeichnet, daß der sich in Längsrichtung erstreckende Schlitz (30) hergestellt wird, indem der Schlitz (30) in einer Anzahl von sich in Längsrichtung erstreckenden Schlitzabschnitten spanabhebend hergestellt wird, wobei ein Steg (32) aus Schaufelmaterial zwischen benachbarten Abschnitten stehengelassen wird.

7. Verfahren nach Anspruch 1, dadurch gekennzeichnet, daß der sich in Längsrichtung erstreckende Schlitz (30) gebildet wird durch spanabhebendes Herstellen des Schlitzes (30) mit einer Breite in bezug auf die Tiefe der ersten Nutoberfläche (14) innerhalb von 0,076 mm (3 mils) von der äußeren Oberfläche (16), wodurch der Rand des verbleibenden Materials in der Dicke 0,076 mm (3 mils) nicht überschreitet.

8. Verfahren nach Anspruch 1, gekennzeichnet durch Gießen des Schaufelblattrohlings (10) mit einem stufenförmigen Übergang ab einem hypothetischen durchgehenden Fortsatz (42) der Schaufelblattoberfläche (16) an der Stelle der Nut (12).

9. Verfahren nach Anspruch 8, gekennzeichnet durch Herstellen des Schaufelblattrohlings (10) mit einem stufenweisen Übergang zwischen 0,05 und 0,25 mm (2 und 10 mils).

FIG. 1

FIG. 2

EP 0 326 508 B1

FIG. 3

FIG. 4

FIG. 5

FIG. 6

EP 0 326 508 B1